# EUROPEAN PATENT APPLICATION

(11) **EP 3 138 596 A1**
(43) Date of publication of application: **08.03.2017**
(21) Application number: 15786742.5
(22) Date of filing: 28.04.2015
(51) Int. Cl.: A61M 5/31, A61M 5/315, A61M 5/20, A61M 5/14

(54) **LENGTH-REDUCING SYRINGE DRIVING DEVICE**

(30) Priority: 29.04.2014 KR 20140051660
(71) Applicant: Choi, Kyu Dong, Seoul 135-969 (KR)
(72) Inventor: Choi, Kyu Dong, Seoul 135-969 (KR)
(74) Representative: Patentanwälte ter Smitten Eberlein Rütten Partnerschaftsgesellschaft
(86) International application number: PCT/KR2015/004218
(87) International publication number: WO 2015/167201

(57) **Abstract**

Disclosed herein is a length-reducing syringe driving device, the length-reducing syringe driving device wherein a drive for syringe device is installed at a syringe device (10), and pushes and drives a piston (12) installed at an injection cartridge (11) which has a discharging outlet (13) at one end thereof.

The length-reducing syringe driving device (100) comprises: a rotation driving part (110) installed at one or more places at an outer sidewall surface at the other end of the injection cartridge (11); and a one-way bending member (120) provided meet to the piston (12) at one end thereof, configured to be bended exclusively to one direction, and having a sidewall surface of a bending , which provide a predetermined range of an independent rotation of an outer cap (700), driven by the rotation driving part (110) and pushing the piston (12).

In the present invention, main elements of the invention are disposed at an outer sidewall surface of an injection cartridge and the entire length (L) of a driving device is remarkably reduced when having the same stroke. Therefore, it is to provide a syringe device which is convenient to be carried and to make syringe devices having many different embodiments possible.

## Description

### BACKGROUND OF THE INVENTION

The present invention relates to a length-reducing syringe driving device, more specifically the length-reducing syringe driving device which is disposed at a syringe device 10 and discharges a small amount of an injection by pushing a piston 12 installed at an injection cartridge 11 forming a discharging outlet at one end thereof, comprising:

a rotation driving part 110 provided at more than one place at outer sidewall surface of the other end of the injection cartridge 11; and a one-way bending member 120 wherein one end thereof is contacted to the piston 12, is configured to be bent exclusively to one direction, wherein a sidewall surface to a bending direction is driven by the rotation driving part 110, thereby pushing and driving the piston 12.

Generally, types of insulin used to treat diabetic metabolic disease may be classified into mealtime insulin and basal insulin. The meal insulin is usually injected into a body three times a day when a diabetic patient is in a fasting state before meals so as to prevent the level of blood sugar of the diabetic patient from sharply rising after meal. The basal insulin acts in a body for 24 hours to keep the function of insulin continuous and steady.

Diabetes is a medical condition in which a body does not produce enough insulin or does not use insulin properly, such that blood sugar, namely glucose, is not converted into energy. Therefore, it is very important to inject a fixed amount of insulin to this diabetic patient at a fixed time. Since insulin should be injected at the fixed time even though that blockage might arise while injecting the insulin, it is considered that injecting the insulin at the fixed time is far more important than injecting the insulin with the fixed amount.

Hereupon, diabetic patients are making use of a portable injection syringe device so that they can be injected with insulin, a medicine for diabetics, at any time while having a regular daily life. For this purpose, the injection syringe device includes a cartridge installation part where a cartridge filled with insulin is installed and a pusher which injects insulin to the patient by pushing the cartridge and discharging the insulin through a discharging outlet.

This type of portable injection syringe devices is usually called "Insulin Pen", and there are lots of kinds of products and models being used. Most insulin pens are classified into two categories, disposable pens and recycling pens. An insulin pen has advantages in that it is easy and convenient to inject insulin when a patient is out in comparison to an ordinary insulin syringe and precise amount of insulin can be injected by simply manipulating a dial. As for patent documents, there are U.S. Patent Registration No. 5,308,340 (Title of the invention: MULTIPLE DOSE INJECTION PEN), which discloses a method of injecting insulin manually, and U.S. Patent Registration No. 6,942,646 (Title of the invention: PEN-TYPE INJECTOR HAVING AN ELECTRONIC CONTROL UNIT), which disclose a method in which insulin is automatically injected.

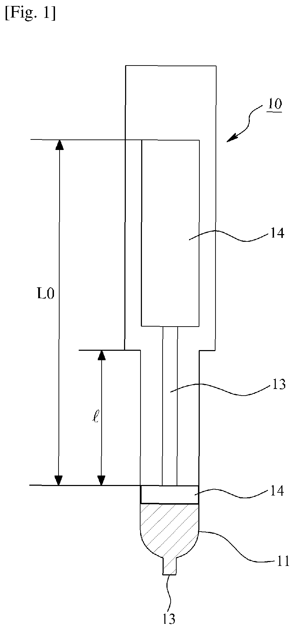

However, no matter they are operated manually or automatically, existing syringe devices, shown in FIG. 1, use a driving device having a stem as an element for operating a piston 12 when it needs a predetermined length of a stroke (*l*) to inject medication in the injection cartridge 11, such that the total length of the driving device (L0) should be at least twice longer than the predetermined length of the stroke (/) above. (That is, L0>**2*l***)

Accordingly, it is structurally impossible, as for existing syringe devices, to reduce their total length, such that it was inconvenient for carrying and there is some limitation to make its structure of the shape.

### SUMMARY OF THE INVENTION

An objective of the present invention is to provide a length-reducing syringe driving device, by solving the problems described in the above. The length-reducing syringe driving device has a simple but efficient structure, wherein main elements of the invention are disposed at an outer sidewall surface of an injection cartridge and the entire length (L) of a driving device is remarkably reduced when having the same stroke. Therefore, it is to provide a syringe driving device which is convenient to be carried and to make many different embodiments of syringe devices possible.

To achieve the objective described in the above, a length-reducing syringe driving device of the present invention, which is installed to a syringe device 10, wherein a predetermined amount of contents is discharged by pushing a piston 12 which is coupled at an injection cartridge 11 having a discharging outlet 13 at one end thereof, comprises:

a rotation driving part 110 installed at more than one place at an outer sidewall surface of the other end of the injection cartridge 11; and a one-way bending member 120, with one end thereof coupled to the piston 12, wherein a sidewall surface to bending direction can be driven by the rotation driving part 110 and pushes the piston 12.

In addition, the length-reducing syringe driving device comprises a separation preventing member 140 which is provided at an outer sidewall surface of the one-way bending member 120 contacted to the rotation driving part 110 and thereby prevents the one-way bending member 120 from being separated. The one-way bending member 120 is characterized to be provided such that a sidewall surface to the bending direction 'a' is contacted to an inner sidewall surface (11a) of the injection cartridge 11.

In addition, the one-way bending member 120 is characterized
to have bending links 121 connected each other.

In addition, the bending links 121 are characterized to comprise:
a protruding connection link 122 wherein a first connecting shaft 123 is provided at one side thereof;
a second connecting shaft 124 which is provided at the other side thereof and connected to the first connecting shaft 123 of the bending links 121;
a driving protrusion 125 provided at a sidewall surface to the bending direction 'a'; and
a bending preventing protrusion 126 which is provided at a sidewall surface to the non-bending direction 'b', and has protrusions 127, 127' respectively at one side direction and at the other side direction.

The rotation driving part 110 is characterized to further comprise a drive gear 111 wherein the driving protrusion 125 is coupled and driven.

In addition, it is characterized to comprise a piston pressing member 130 which is connected to one end of the one-way bending member 120.

The present invention has an advantage in that it is convenient to be carried by remarkably reducing the entire length (L) of a driving device when main elements of the invention are disposed at an outer sidewall surface of an injection cartridge so as to have the same stroke, and thereby making many different embodiments of the syringe device possible.

### BRIEF DESCRIPTION OF THE DRAWINGS

FIG. 1 is a mimetic diagram illustrating the configuration of a length-reducing syringe driving device according to an exemplary embodiment of the existing invention;
FIG. 2 is a mimetic diagram illustrating a configuration of a length-reducing syringe driving device in which a length-reducing syringe driving device is applied according to an exemplary embodiment of the present invention.
FIG. 3 is a mimetic diagram illustrating an operational state of a short length driver for a length-reducing syringe driving device according to an exemplary embodiment of the present invention.
FIG. 4 is a view illustrating a configuration of main parts of a short length driver for a length-reducing syringe driving device according to an embodiment of the present invention.
FIG. 5 is a view illustrating a configuration and an operational state of a one-way bending member of a length-reducing syringe driving device according to an exemplary embodiment of the present invention.
FIG. 6 is a view illustrating a configuration a bending link composed of a one-way bending member of a length-reducing syringe driving device according to an exemplary embodiment of the present invention.

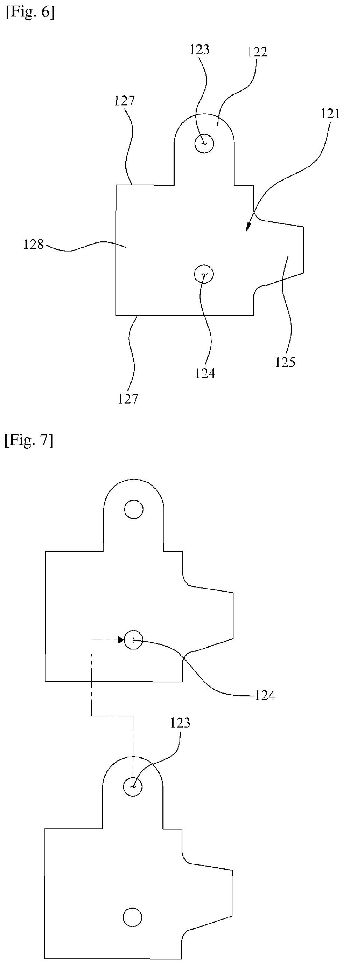

FIG. 6 is a mimetic diagram illustrating a configuration a bending link composed in a one-way bending member of a length-reducing syringe driving device according to an exemplary embodiment of the present invention.

### DETAILED DESCRIPTION OF PREFERRED EMBODIMENTS

Hereinafter, a length-reducing syringe driving device according to exemplary embodiments of the present invention will be described in detail with reference to the accompanying drawings. The same reference numerals provided in the drawings indicate the same members. In describing the present invention, detailed descriptions regarding to the technology or the configuration of related prior arts are omitted not to make points of the present invention unclear.

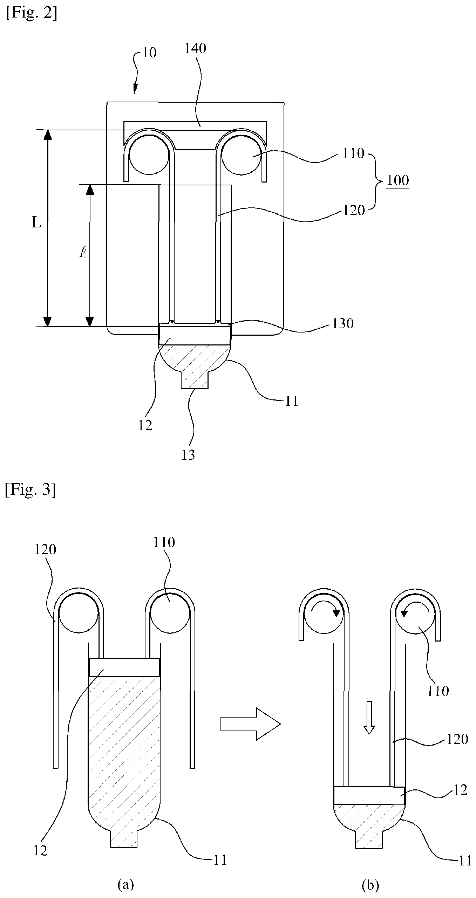

The length-reducing syringe driving device according to an exemplary embodiment of the present invention, shown in Fig. 2, is driven to discharge a predetermined amount of contents by pushing a piston 12 provided at an injection cartridge 11 which comprises a discharging outlet 13 at one end thereof.

For the operation described in the above, a length-reducing syringe driving device according to an exemplary embodiment of the present invention as shown in Fig. 2 comprises a rotation driving part 110 and a one-way bending member 120.

First, a rotation driving part 110 is to be described. The rotation driving part 110 as shown in Fig.2 is provided at more than one place of an outer sidewall surface of the other end of the injection cartridge 11, and has a function of driving the one-way bending member 120 by a rotating operation. In this case, the rotation driving part 110 can be driven by being installed at one place of the outer sidewall surface of one end of the injection cartridge 11. However, to make a stable driving under the balance with a driving force, the rotation driving part 110 is preferred to be provided at both sides corresponding to the outer sidewall surface of the other end, as shown in Fig. 2. In addition, the most preferred installing shape is that rotation driving part 110 is installed evenly spaced (that is, with 120° apart) at three places of the outer sidewall surface of the other end of the injection cartridge 11.

Meanwhile, the rotation driving part 110 may have an exemplary embodiment possible, wherein a manually controlling handle, an electric motor, an oil pressure rotation means and more can be included.

In addition, when two or more of the rotation driving parts 110 are installed, each drive of rotation driving parts 110 is preferred to be synchronized and concurred. For this, it is preferred that a sensor, which measures a driving level of the rotation driving part 110 respectively and a control part which feedbacks the measurement of the sensor and controls the drive of the rotation driving part, should be included therein. Meanwhile, in order to concur and synchronize each drive of rotation driving parts 110, the rotation driving part 110 is also possible to comprise a driving means and a driving delivery means which is mechanically connected with the driving means and is respectively driven.

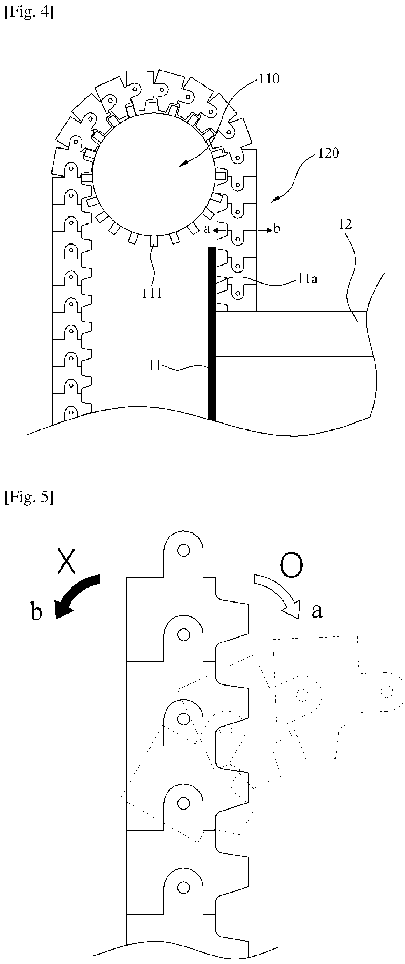

Meanwhile, the rotation driving part 110 can have many different shapes according to the structure of the one-way bending member 120. As for one exemplary embodiment, when configured to include a driving protrusion 125 as shown in FIG.4, the one-way bending member 120 is preferred to further include a driving gear 111 wherein the driving protrusion 125 is coupled and driven.

Next, the following is a detailed description of a one-way bending member 120. The one-way bending member 120 has one end contacted to the piston 12 as shown in FIG. 2, and is bent exclusively to a bending direction 'a' as shown in FIG. 5, such that a sidewall surface to the bending direction 'a' is driven by the rotation driving part 110 and pushes the piston 12 to drive.

In this case, it is preferable that the one-way bending member 120, as shown in FIG. 4, should be provided at an inner sidewall surface 11a of the injection cartridge 11 in order to the sidewall surface of the bending direction 'a' is contacted. In other words, when the one-way bending member 120 delivers a compression force to the piston 12 by the drive of the rotation driving part 110, the one-way bending member 120 may have a deformation of bending to a side direction. However, the one-way bending member 120 has a configuration of bending exclusively to a bending direction 'a', not to the non-bending direction 'b'. Therefore, when a sidewall surface to the bending direction 'a' is provided to meet an inner sidewall surface 11 a as described in the above, the bending to the bending direction 'a' is prevented by the inner sidewall surface, and thus, the one-way bending member 120 is able to deliver the drive force of the rotation driving part 110 to the piston 12 effectively and precisely without being bent or twisted.

Meanwhile, the one-way bending member 120 is preferred to include a piston pressing member 130 at one end thereof, such that the driving force of the one-way bending member 120 can be delivered evenly to the piston 12.

It is possible that the one-way bending member 120 has many different exemplary embodiments, and as an example, the one-way bending member 120 is preferred to have bending links 121 connected each other as shown in FIG. 4 and 5.

In this case, the bending links 121, as shown in FIG. 6, are preferably configured to comprise: a protruding connection link 122 having a first connecting shaft 123 provided at one side thereof; a second connecting shaft 124 which is provided at the other side thereof and connected to the first connecting shaft 123 of the bending links 121, as shown in FIG. 7; a driving protrusion 125 provided at a side to the bending direction 'a'; and a bending preventing protrusion 126 which is provided to a non-bending direction 'b' and where protrusions 127, 127' are provided respectively at one side direction and the other side direction.

In other words, as shown as a dotted line in FIG.5, the bending links 121 connected each other through the first connecting shaft 123 and the second connecting shaft 124 can be freely bent to the bending direction 'a'. On the other hand, to the non-bending direction 'b', the protrusions 127, 127'of the bending preventing protrusion 126 provided respectively at the bending links 121 contact each other and interfere with bending, thereby preventing bending to the non-bending direction `b'.

In this case, as described previously, the rotation driving part 110 further comprises a drive gear 111 which is driven by being coupled to the driving protrusion 125.

Meanwhile, the present invention preferably further comprises a separation preventing member 140 which is provided at an outer sidewall surface of the one-way bending member 120 contacted to the rotation driving part 110, and prevents separation of the one-way bending member 120, such that the one-way bending member 120 can be prevented from being separated from the rotation driving part 110 while the rotation driving part 110 delivers the driving force to the one-way bending member 120.

Hereafter, an operation of a length-reducing syringe driving device according to an exemplary embodiment of the present invention will be described.

First, as shown in (a) of FIG. 3, an injection cartridge 11 is installed at a syringe device 10 such that a one-way bending member 120 (or a piston pressing member 130 if installed) can contact a piston 12.

Next, when a rotation driving part 110 operates, the one-way bending member 120 (or a piston pressing member 130 if installed) presses the piston 12 and then descends, as shown in (b) of FIG.3, thereby causing an injection to be discharged through a discharging outlet 13.

According to the above configuration, main elements of the invention are disposed at an outer sidewall surface of an injection cartridge, such that the entire length (L) of the driving device is long enough for the diameter of the rotation driving part 110 to be added to the stroke (/) when having the same stroke (/). Therefore, the entire length (L0) of a driving device is remarkably reduced in comparison to the length of an existing inventions which need twice long length or longer length than the stroke (*l*), thereby achieving an advantage that the present invention is convenient to be carried and has many different embodiments of a syringe device possible as well.

As described above, exemplary embodiments of the present invention are disclosed. Although specific terms have been used herein, these are only intended to describe the present invention and are not intended to limit the meanings of the terms or to restrict the scope of the present invention as disclosed in the accompanying claims. Therefore, those skilled in the art will appreciate that various modifications and other equivalent embodiments are possible from the above embodiments. Therefore, the scope of the present invention should be defined by the technical spirit of the accompanying claims.

## Claims

1. A length-reducing syringe driving device,
wherein a syringe driving device installed at a syringe device (10) pushes and drives a piston (12) installed at an injection cartridge (11) having a discharging outlet (13) at one end thereof, comprising:
a rotation driving part (110) installed at one or more places at an outer sidewall surface at the other end of the injection cartridge (11); and
a one-way bending member (120) provided meet to the piston (12) at one end thereof, configured to be bended exclusively to one direction, and having a sidewall surface to a bending direction thereof driven by the rotation driving part (110) and pushing the piston (12)

2. The length-reducing syringe driving device of claim 1, further comprising:
a separation preventing member (140) provided at an outer sidewall surface of the one-way bending member (120) contacted to the rotation driving part (110) and preventing the separation of the one-way bending member (120),
wherein the one-way bending member (120) has a configuration in which a sidewall to the bending direction 'a' thereof is contacted to an inner sidewall surface (11a).

3. The length-reducing syringe driving device of claim 2,
wherein the one-way bending member (120) is configured in a way that bending links (121) are connected each other.

4. The length-reducing syringe driving device of claim 3,
wherein the bending links (121) comprises:
a protruding connection link (122) wherein a first connecting shaft (123) is provided at one side thereof;
a second connecting shaft (124) provided at the other side thereof and connected with the first connecting shaft (123) of the bending links (121);
a driving protrusion (125) provided to a bending direction 'a'; and
a bending preventing protrusion (126) provided to a non-bending direction 'b' and forming protrusions (127, 127') respectively at the one side and the other side direction,
wherein the rotation driving part (110) further comprises a drive gear (111) which is coupled and driven by a driving protrusion (125)

5. The length-reducing syringe driving device of claim 4, further comprising a piston pressing member (130) which is connected at one end of the one-way bending member (120).
